(19) Europäisches Patentamt
European Patent Office
Office européen des brevets

(11) EP 3 950 804 A1

(12) EUROPEAN PATENT APPLICATION
published in accordance with Art. 153(4) EPC

(43) Date of publication:
09.02.2022 Bulletin 2022/06

(21) Application number: 21741617.1

(22) Date of filing: 12.01.2021

(51) International Patent Classification (IPC):
C08K 5/11 (2006.01) C08K 5/00 (2006.01)
C08L 101/00 (2006.01)

(52) Cooperative Patent Classification (CPC):
C08K 5/00; C08K 5/11; C08L 101/00

(86) International application number:
PCT/KR2021/000388

(87) International publication number:
WO 2021/145643 (22.07.2021 Gazette 2021/29)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 15.01.2020 KR 20200005440

(71) Applicant: LG CHEM, LTD.
Yeongdeungpo-gu,
Seoul 07336 (KR)

(72) Inventors:
- KIM, Hyun Kyu
Daejeon 34122 (KR)
- JEONG, Seok Ho
Daejeon 34122 (KR)
- CHOI, Woo Hyuk
Daejeon 34122 (KR)
- MOON, Jeong Ju
Daejeon 34122 (KR)
- KIM, Joo Ho
Daejeon 34122 (KR)

(74) Representative: Goddar, Heinz J.
Boehmert & Boehmert
Anwaltspartnerschaft mbB
Pettenkoferstrasse 22
80336 München (DE)

# (54) CITRATE-BASED PLASTICIZER COMPOSITION AND RESIN COMPOSITION COMPRISING SAME

(57) The present invention relates to a plasticizer composition, including a lower alkyl-based citrate and a higher alkyl-based citrate at the same time, as citrate, wherein the ratio of a hybrid type to a non-hybrid type and the ratio of the lower alkyl groups to the higher alkyl groups are controlled so that effects are achieved. When applying the plasticizer composition to a resin, stress resistance and mechanical properties can be maintained at an equal or higher level, the migration and volatile loss properties and the plasticization efficiency can be balanced, and the light resistance and heat resistance can be remarkably improved.

EP 3 950 804 A1

**Description**

## TECHNICAL FIELD

### Cross-reference to Related Applications

The present application claims the benefit of priority based on Korean Patent Application No. 10-2020-0005440, filed on 15 January 2020, the entire disclosure of which is incorporated as part of the specification.

### Technical Field

The present invention relates to a citrate-based plasticizer composition comprising a hybrid citrate of lower and higher alkyl radicals of components therein, and a resin composition comprising the same.

## BACKGROUND ART

Generally, a plasticizer forms an ester corresponding to the plasticizer through a reaction between an alcohol and a polycarboxylic acid such as phthalic acid or adipic acid. Also, there has been continuing research on compositions of plasticizers that can replace phthalate-based plasticizers such as terephthalate-based, adipate-based, and other polymer-based plasticizers in consideration of domestic and international regulations on phthalate-based plasticizers which are harmful to human bodies.

Meanwhile, there is an increasing demand for environmentally friendly products relating to flooring materials, wallpaper, soft and hard sheets, etc. obtained in the plastisol industry, the calendering industry, the extruding/injecting compound industry, etc., and to reinforce quality characteristics, processability and productivity of each end product for such environmentally friendly products, suitable plasticizers have to be used depending on discoloration, migration, mechanical properties, etc.

Depending on the characteristics required by industry in the various areas of use, such as tensile strength, an elongation rate, light resistance, migration properties, gelling properties, an absorption rate, etc., a PVC resin is mixed with a supplementary material such as plasticizers, fillers, stabilizers, viscosity reducing agents, dispersants, antifoaming agents, foaming agents, and the like.

For example, among the plasticizer compositions applicable to PVC, when di(2-ethylhexyl) terephthalate (DEHTP), which is relatively cheap and most widely used, was applied, hardness or sol viscosity was high, the absorption rate of the plasticizer was relatively slow, and migration properties and stress migration properties were poor.

To improve these properties, it may be considered to use a product of transesterification with butanol as a plasticizer composition containing DEHTP. However, using the product improves plasticization efficiency, but results in poor heat loss or thermal stability, and slightly degraded mechanical properties, and thus there is a need for improvement in physical properties. In general, there is no solution as of now except to adopt a way to make up this through a combination with other secondary plasticizers.

However, using a secondary plasticizer has the following drawbacks: it is difficult to predict changes in physical properties; product unit prices may increase; improvements in physical properties are not apparent except under certain cases; and unexpected problems, such as a problem of compatibility with resin, occur.

Also, to improve the poor migration and weight loss properties of the DEHTP product, using a material being a trimellitate-based product, such as tri(2-ethylhexyl) trimellitate or triisononyl trimellitate, improves the migration or weight loss properties, but results in poor plasticization efficiency, and thus, there is a problem that a considerable amount should be added to provide an appropriate plasticizing effect for the resin. Moreover, the unit price of the material is relatively high, and thus, commercialization is not possible.

Accordingly, there is a need to develop a product for solving environmental issues of the phthalate-based product as an existing product, or a product having improved physical properties of the eco-friendly product to improve the environmental issues of the phthalate-based product.

## DISCLOSURE OF THE INVENTION

## TECHNICAL PROBLEM

The present invention is to provide a plasticizer composition, which contains citrates, in which lower and higher alkyl radicals are appropriately controlled and bound, and thus can improve mechanical properties and stress resistance compared to conventional plasticizers and at the same time, allow balanced improvements in the migration resistance and volatile loss properties and the plasticization efficiency, and improve heat resistance (retention properties).

## TECHNICAL SOLUTION

To solve the above-described tasks, according to an embodiment of the present invention, there is provided a plasticizer composition including a citrate-based composition containing three or more citrates represented by the following Formula 1, wherein an alkyl group of the citrate is derived from a C4 alcohol and a C7 alcohol, the C4 alcohol includes one or more selected from the group consisting of n-butanol and iso-butanol, and the C7 alcohol includes one or more selected from n-heptyl group or a branched heptyl group:

[Formula 1]

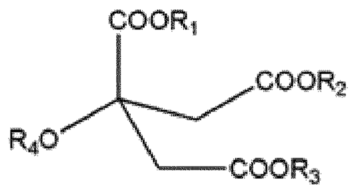

In Forumal 1, $R_1$ to $R_3$ are each independently an alkyl group having four or seven carbon atoms, and $R_4$ is hydrogen or an acetyl group.

To solve the above-described tasks, according to another aspect of the present invention, there is provided a resin composition including: 100 parts by weight of a resin; and 5 to 150 parts by weight of the above-described plasticizer composition.

The resin may be one or more selected from the group consisting of a straight vinyl chloride polymer, a paste vinyl chloride polymer, an ethylene vinyl acetate copolymer, an ethylene polymer, a propylene polymer, polyketone, polystyrene, polyurethane, natural rubbers, and synthetic rubbers.

## ADVANTAGEOUS EFFECTS

The plasticizer composition according to an embodiment of the present invention, when being used in a resin composition, can improve mechanical properties and stress resistance compared to conventional plasticizers and at the same time, allow balanced improvements in the volatile loss properties and the plasticization efficiency, and improve the migration resistance and retention properties.

## MODE FOR CARRYING OUT THE INVENTION

Hereinafter, The terms or words used in the description and claims shall not be interpreted as being limited to ordinary or dictionary meanings and the terms or words should be interpreted as meanings and concepts consistent with the technical idea of the present invention, based on the principle that an inventor may properly define the concept of a term to explain his own invention in the best way.

### Definition of terms

The term "composition" used in the description includes a mixture of materials including a corresponding composition as well as a reaction product and a decomposition product produced from the materials of the corresponding composition.

The term "straight vinyl chloride polymer" used in the description means a kind of a vinyl chloride polymer which is polymerized by suspension polymerization or bulk polymerization. This polymer has a porous particle shape having a large number of pores, a size of the particle in the range of tens to hundreds of micrometers, no cohesion, and excellent flowability.

The term "paste vinyl chloride polymer" used in the description means a kind of a vinyl chloride polymer which is polymerized by microsuspension polymerization, micro-seeded polymerization or emulsion polymerization. This polymer has a non-porous, minute and dense particle shape having a size in the range of tens to thousands of nanometers, cohesion, and inferior flowability.

The terms "comprising", "having" and the derivatives thereof, whether particularly disclosed or not, are not intended to preclude the presence of any additional components, steps, or procedures. In order to avoid any uncertainty, all compositions claimed by using the term "comprising" may include any additional additives, auxiliaries, or compounds, including a polymer or any other materials, unless otherwise described to the contrary. In contrast, the term "consisting

essentially of" excludes unnecessary ones for operation and precludes any other components, steps or procedures from the scope of any continuous description. The term "consisting of" precludes any components, steps or procedures, which are not specifically described or listed.

**Measurement method**

In the present description, analysis of the contents of components in a composition is performed by gas chromatography measurement using a gas chromatography instrument of Agilent Co. (product name: Agilent 7890 GC, column: HP-5, carrier gas: helium (flow rate 2.4 mL/min), detector: F.I.D, injection volume: 1 μL, initial value: 70°C/4,2 min, terminal value: 280°C/7.8 min, program rate: 15°C/min).

In the description, "hardness" means shore hardness (Shore "A" and/or Shore "D") at 25°C, measured under conditions of 3T 10s by using ASTM D2240, and may be an index for evaluating plasticization efficiency. The lower the hardness is, the better the plasticization efficiency is.

In the description, "tensile strength" is measured according to ASTM D638. After pulling at a cross head speed of 200 mm/min (1T) by using a test instrument of U.T.M (manufacturer; Instron, model name; 4466), a position where a specimen is cut is measured, and the tensile strength is calculated by the following Equation 1.

[Equation 1]

Tensile strength ($kgf/cm^2$) = load value (kgf) / thickness (cm) x width (cm)

In the description, "elongation rate" is measured according to ASTM D638. After pulling at a cross head speed of 200 mm/min (1T) by using the U.T.M, a position where a specimen is cut is measured, and the elongation rate is calculated by the following Equation 2.

[Equation 2]

Elongation rate (%) = length after elongation / initial length x 100

In the description, "migration loss" is measured according to KSM-3156. A specimen with a thickness of 2 mm or more is obtained, and glass plates are attached onto both sides of the specimen and a load of 1 $kgf/cm^2$ is applied. The specimen is stood in a hot air circulation oven (80°C) for 72 hours and is taken out and cooled at room temperature for 4 hours. After that, the glass plates attached onto both sides of the specimen are removed, and the weights of the glass plates and the specimen plate before and after being left standing in the oven are measured. The migration loss is calculated by the following Equation 3.

[Equation 3]

Migration loss (%) = {[(initial weight of specimen at room temperature) - (weight of specimen after being left standing in oven)] / (initial weight of specimen at room temperature)} x 100

In the description, "volatile loss" is obtained by processing the specimen at 80°C for 72 hours, and measuring the weight of the specimen.

[Equation 4]

Volatile loss (wt %) = {[(weight of initial specimen - weight of specimen after processing)] / (weight of initial specimen)} x 100

The condition details such as temperature, rotational speed, and time among various measurement conditions may be somewhat varied by cases, and in different cases, the measurement method and the conditions thereof will be specified.

Hereinafter, the present invention will be described in more detail to help an understanding of the present invention.

According to an embodiment of the present invention, a plasticizer composition includes a citrate-based composition containing three or more citrates represented by the following Formula 1, wherein an alkyl group of the citrate is derived from a C4 alcohol and a C7 alcohol, the C4 alcohol includes one or more selected from the group consisting of n-butanol and iso-butanol, and the C7 alcohol includes one or more selected from n-heptyl group or a branched heptyl group.

[Formula 1]

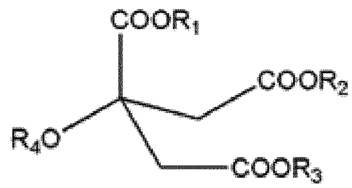

In Forumal 1, $R_1$ to $R_3$ are each independently an alkyl group having four or seven carbon atoms, and $R_4$ is hydrogen or an acetyl group.

Specifically, the citrate-based composition may include: a lower alkyl-based citrate including a lower non-hybrid citrate having a C4 alcohol-derived alkyl group, and a lower hybrid citrate having a C4 alcohol-derived alkyl group and a C7 alcohol-derived alkyl group, in which the C4 alcohol-derived alkyl groups are more than the C7 alcohol-derived alkyl groups; and a higher alkyl-based citrate including a higher hybrid citrate having a C4 alcohol-derived alkyl group and a C7 alcohol-derived alkyl group, in which the C7 alcohol-derived alkyl groups are more than the C4 alcohol-derived alkyl groups, and a higher non-hybrid citrate having a C7 alcohol-derived alkyl group.

More specifically, according to an embodiment of the present invention, the citrate-based composition contained in the plasticizer composition contains a total of four types of citrates, which can be largely classified into higher alkyl-based citrates having two or more C7 alkyl groups bound (Formulas 1-4 to 1-6) and lower alkyl-based citrates having two or more C4 alkyl groups bound (Formulas 1-1 to 1-3). Also, the lower alkyl-based citrates can be subdivided into a lower non-hybrid citrate (Formula 1-1), in which the C4 alkyl groups are bound to all the three ester groups, and lower hybrid citrates (Formulas 1-2 and 1-3), in which the C4 alkyl groups are bound to two ester groups. Similarly, the higher alkyl-based citrates can be divided into a higher non-hybrid citrate (Formula 1-6) and higher hybrid citrates (Formulas 1-4 and 1-5). In detail, for the citrates represented by Formulas 1-3 and 1-5, optical isomers may exist due to the presence of a chiral carbon, but in the present specification, the optical isomers are not treated separately as different compounds.

Herein, the term "having ... alky groups bound" may mean "having ... alkyl groups bound to three ester groups of the citrate.

Also, the term "non-hybrid" or "hybrid" distinguishes, based on the alkyl groups bound to three esters, whether alkyl radicals having the same number of carbon atoms only are bound or alkyl radicals having a different number of carbon atoms are mixed to be bound. When alkyl radicals having the same number of carbon atoms are bound to all the three ester groups, it may be referred to as "non-hybrid." When alkyl radicals having a different number of carbon atoms are mixed to be bound to the three ester groups, it may be referred to as "hybrid." In detail, the hybrid and non-hybrid are distinguished based on the number of carbon atoms. For example, when only heptyl groups having the same number of carbon atoms are bound, even if the n-pentyl group and a branched heptyl group are mixed, the number of carbon atoms thereof is identical, and thus it means "non-hybrid" in the present specification. The same is true for butyl groups.

Formulas 1-1 to 1-6 each representing the higher alkyl-based citrates and the lower alkyl-based citrates in the citrate-based composition are shown as follows:

[Formula 1-1]

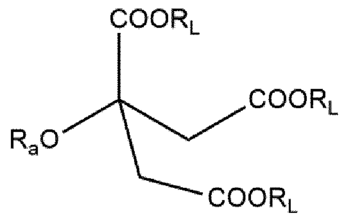

[Formula 1-2]

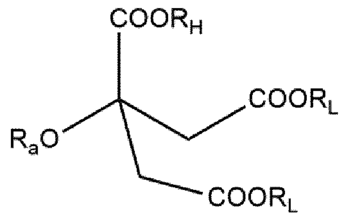

[Formula 1-3]

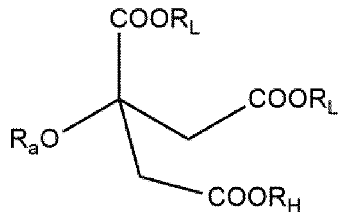

[Formula 1-4]

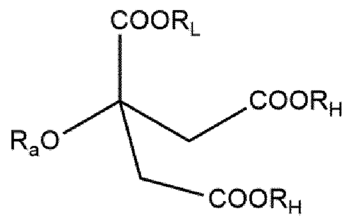

[Formula 1-5]

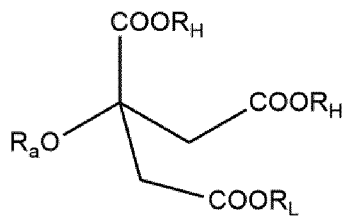

[Formula 1-6]

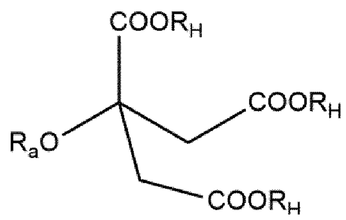

In Formulas 1-1 to 1-6, $R_L$ is n-butyl group or iso-butyl group, $R_H$ is n-heptyl group or a branched heptyl group, and $R_a$ is hydrogen or an acetyl group.

The plasticizer composition may be a product produced by direct esterification of a mixture of citric acid or a citric acid derivative and an alcohol having an alkyl group having four or seven carbon atoms, or by transesterification of a citrate having an alkyl group having four (or seven) carbon atoms and an alcohol having seven (or four) carbon atoms. Herein, the alcohol to be applied may be a mixture of structural isomers or any single substance. For example, the alcohol having four carbon atoms may be purified n-butanol alone. The alcohol having seven carbon atoms may be n-heptanol or iso-heptanol alone. The alcohol having four carbon atoms may be a mixture of n-butanol and iso-butanol. The alcohol having seven carbon atoms may be a mixture of n-heptanol and a branched heptanol.

The plasticizer according to an embodiment of the present invention includes a total of four types of citrates as described above, and thus excellent effects can be implemented by a combination of alkyl groups suitably bound to each type.

Specifically, the plasticization efficiency and physical properties such as migration/volatile loss properties can be balanced due to the balance of the alkyl groups between the higher non-hybrid citrates and the lower non-hybrid citrates and coexistence of hybrid types in the composition, moreover, characteristics such as the controlled ratio of the lower alkyl groups and the higher alkyl groups among all the alkyl radicals, furthermore, when any one alkyl group is derived from a mixed alcohol, the ratio of certain branched alkyl radicals in the branched alkyl groups. Due to the interaction of the four types of citrates contained in the composition, remarkable improvement in mechanical properties, stress resistance, and retention properties can be achieved.

Thus, a product free from environmental issues of existing phthalate-based products and having more improved volatile loss property can be implemented, and the migration and volatile loss properties of conventional terephthalate-based products can be significantly improved, and a product having greatly improved mechanical properties and stress resistance compared to existing commercial products can be implemented.

To more optimally and preferably achieve the above effects, it may be important to meet the conditions of $R_L$ and $R_H$ defined in Formulas 1-1 to 1-6.

As defined above, $R_L$ and $R_H$ may each be n-butyl group or iso-butyl group, and n-heptyl group or iso-heptyl group. These alkyl radicals are factors that can determine the interaction between each type of citrates contained in the citrate-based plasticizer composition and the weightiness of the entire composition and may play a major role in achieving the effects.

Preferably, $R_L$ is an alkyl group having four carbon atoms, may be n-butyl group or iso-butyl group. Also, $R_H$ is an alkyl group having seven carbon atoms and may be n-heptyl group or iso-heptyl group. The iso-heptyl group may be one selected from the group consisting of 2-methyl hexyl, 3-methyl hexyl, 4-methyl hexyl, 5-methyl hexyl, 2-ethyl pentyl, 3-ethyl pentyl, 4-ethyl pentyl, 2,2-dimethyl pentyl, 2,3-dimethyl pentyl, 2,4-dimehtyl pentyl, 3,3-dimethyl pentyl, 3,4-dimethyl pentyl, and 4,4-dimehtyl pentyl. Preferably, $R_H$ may be n-heptyl group, 2-methyl hexyl, 3-methyl hexyl, 4-methyl hexyl, 5-methyl hexyl group, 2,2-dimethyl pentyl, 2,3-dimethyl pentyl, or 2,4-dimethyl pentyl.

According to an embodiment of the present invention, the C7 alcohol may include essentially n-heptanol. There are various isomers of the C7 alcohol. It may be preferable that n-heptanol among those is included as the C7 alcohol. n-Heptanol is a linear alcohol, and the intermolecular interaction and steric hindrance effect can be appropriately controlled, and thus, the performance of the plasticizer can be improved. This may be caused by a synergistic effect in connection with the fact that 2-ethyl hexanol, a lower alcohol, is a branched alcohol.

Also, the C7 alcohol includes n-heptanol but may include at least one branched alcohol selected from 4-methyl hexanol and 5-methyl hexanol, and n-heptanol may be included in an amount of 50 wt% or more based on the total weight of the C7 alcohol. The C7 alcohol may be used in the form of a mixture of isomers, wherein when n-heptanol is included in an amount of 50 wt% or more in the mixture, the plasticizer performance may become more excellent.

In the plasticizer composition according to an embodiment of the present invention, to further optimize the implementation of the effects according to the present invention, the ratio of each alkyl group may be adjusted. Firstly, the weight ratio of the lower alkyl-based citrate to the higher alkyl-based citrate may be controlled to 80:20 to 1:99, preferably, 80:20 to 5:95, or 80:20 to 10:90, more preferably, 70:30 to 20:80, and even more preferably, 90:10 to 30:70.

By using the lower alkyl-based citrates, which are represented by Formulas 1 to 3, more than the higher alkyl-based citrates (Formulas 4 to 6), the weightiness of the entire plasticizer composition can be controlled, and accordingly, a significant improvement in the performance of the plasticizer composition can be expected.

In more detail, a factor that determines the structural properties of the plasticizer composition according to an embodiment of the present invention is controlling the weight ratio of the non-hybrid citrates represented by Formulas 1-1 and 1-6 to the hybrid citrates represented by Formulas 1-2 to 1-6. The weight ratio of the non-hybrid citrates to the hybrid citrates may be 80:20 to 5:95, preferably, 70:30 to 10:90, and more preferably, 60:40 to 20:80. In the esterification process for preparing the composition, the amount of the product to which the mixed alkyl groups are bound can be controlled by controlling the reaction, and thus, it can play a significant role in achieving the effects.

Likewise, controlling the weight ratio of the higher non-hybrid citrate represented by Formula 6 to the higher hybrid citrates represented by Formulas 4 and 5 among the higher alkyl-based citrates to 80:20 to 20:80, preferably, 70:30 to 30:70, and more preferably, 60:40 to 30:70 may also play a similar role. Thus, it is necessary to note that the effect of the resulting plasticizer composition can be improved by controlling the weight ratio between citrates included in the higher alkyl-based citrates through appropriate control during the reaction.

When the components of the plasticizer composition according to the present invention are composed within the above ranges, considering the equivalent ratio of substances used as reactants, actual yield, conversion rate, etc. of the reaction, productivity in the manufacturing process can be increased, deterioration of mechanical properties such as tensile strength and elongation rate, described above, can be prevented, and a significant improvement in light resistance can be exhibited.

Meanwhile, the substituent defined as Ra in the citrate may be hydrogen or an acetyl group. For improvement and optimization of physical properties of the plasticizer, particularly processability, melting properties, and mechanical properties, such as elongation rate, due to a decrease in plasticization efficiency, hydrogen may be more preferable. Also, when the substituent is an acetyl group, it is necessary to take into account that it may be difficult to rule out the problem of lowering economic efficiencies such as an increase in cost due to production problems such as the addition of production processes and treatment facilities due to the generation of waste acetic acid.

The method for preparing the plasticizer composition according to an embodiment of the present invention is a well-known method in the art, and any methods can be applied without specific limitation, so long as the above-described plasticizer composition is prepared.

For example, the composition can be prepared through direct esterification of citric acid or an anhydride thereof and two or more kinds of alcohols. Also, the composition can be prepared through transesterification of citrate and one kind of alcohol.

The plasticizer composition according to an embodiment of the present invention is a material prepared by appropriately performing the esterification. Thus, as long as the above-described conditions are fulfilled, in particular, as long as the ratio of the branched alcohol in the isomers-mixed alcohol is controlled, there is no particular limitation on the preparation methods.

For example, the direct esterification may be performed by a step for introducing citric acid and two or more kinds of alcohols, adding a catalyst and reacting under a nitrogen atmosphere; a step for removing unreacted alcohols and neutralizing unreacted acid; and a step for dehydrating by distillation in a reduced pressure and filtering.

The alcohol may be a monoalcohol having an alkyl group corresponding to $R_H$ and $R_L$ in the Formulas 1-1 to 1-6. The weight ratio of a monoalcohol having the alkyl group of $R_L$ and a monoalcohol having the alkyl group of $R_H$ may function as an important factor for determining the ratio of components in the prepared composition. For example, the weight ratio of the C4 alcohol and the C7 alcohol may be 90:10 to 10:90, preferably, 80:20 to 10:90, more preferably, 75:25 to 10:90, or 70:30 to 10:90, and even more preferably, 60:40 to 10:90.

The alcohol may be used in a range of 150 to 500 mol%, 200 to 400 mol%, 200 to 350 mol%, 250 to 400 mol%, or 270 to 330 mol% based on 100 mol% of the acid. By controlling the content of this alcohol, the component ratio in the final composition can be controlled.

The catalyst may be, for example, an acid catalyst such as sulfuric acid, hydrochloric acid, phosphoric acid, nitric acid, paratoluene sulfonic acid, methane sulfonic acid, ethane sulfonic acid, propane sulfonic acid, butane sulfonic acid, and alkyl sulfate, a metal salt such as aluminum lactate, lithium fluoride, potassium chloride, cesium chloride, calcium chloride, iron chloride, and aluminum phosphate, a metal oxide such as heteropoly acids, natural/synthetic zeolites, cation and anion exchange resins, and an organometal such as tetra alkyl titanate and polymers thereof. A specific example of the catalyst may be tetra alkyl titanate. Preferably, as an acid catalyst having a low active temperature, paratoluene sulfonic acid, methane sulfonic acid, or the like may be appropriate.

The amount used of the catalyst may be different according to the kind. For example, a homogeneous catalyst may be used in an amount of 0.01 to 5.0 wt%, 0.01 to 3.0 wt%, 1.0 to 5.0 wt% or 2.0 to 4.0 wt% based on total 100 wt% of the reactants, and a heterogeneous catalyst may be used in an amount of 5 to 200 wt%, 5 to 100 wt%, 20 to 200 wt% or 20 to 150 wt% based on the total amount of the reactants.

Herein, the reaction temperature may be in a range of 180 to 280°C, 200 to 250°C, or 210 to 230°C.

As another example, the transesterification may be a reaction of citrate and alcohols having alkyl radicals having a different number of carbon atoms from the alkyl radicals of the citrate (for the citrate having the higher alkyl group bound, the lower alkyl alcohols; for the citrate having the lower alkyl group, the higher alkyl alcohols). Herein, the alkyl groups of the citrate and the alcohol may be crossed over each other.

"Transesterification" used in the present invention means the reaction of an alcohol and an ester to interchange an alkyl of the ester with an alkyl of the alcohol:

In the case of the citrates included in the plasticizer composition according to the present invention, according to the ester group bonding position, when two ester groups are interchanged, and when one ester group is interchanged, three types may be formed respectively, and accordingly, in the final composition, a mixture of up to 8 compounds (including structural isomers and optical isomers) may be present.

In addition, the transesterification has the advantage of not generating wastewater problems when compared with the esterification between acid-alcohol.

The composition ratio of the mixture prepared through the transesterification may be controlled according to the addition amount of the alcohol. The addition amount of the alcohol may be 10 to 200 parts by weight, specifically, 20 to 150 parts by weight, more particularly, 30 to 120 parts by weight based on 100 parts by weight of the citrate compound. For reference, a determinant for the ratio of components in the final composition may be the addition amount of the alcohol as in the direct esterification.

That is, in the citrate-based composition, since the mole fraction of the citrate participating in the transesterification can increase according to the increase of the addition amount of the alcohol, the content of citrate, which is the product, in the mixture can increase, and correspondingly, the content of the unreacted citrate can tend to decrease.

According to an embodiment of the present invention, the molar ratio of the reactants, citrate and alcohols may be, for example, 1:0.005 to 1:10, 1:0.05 to 1:8, or 1:0.1 to 1:6, and within this range, processing efficiency and economic feasibility can be excellent and a plasticizer composition capable of achieving the above-described effects can be obtained.

According to an embodiment of the present invention, the transesterification may be performed at a temperature of 120 to 190°C, preferably, 135 to 180°C, more preferably, 141 to 179°C for 10 minutes to 10 hours, preferably, 30 minutes to 8 hours, more preferably, 1 to 6 hours. Within the temperature and time ranges, the ratio of components in the final plasticizer composition can be efficiently controlled. Herein, the reaction time may be calculated from a point when the temperature of the reactants is elevated and arrives at the reaction temperature.

The transesterification may be performed under an acid catalyst or a metal catalyst. Herein, the reaction time can be shortened.

The acid catalyst may be, for example, sulfuric acid, methanesulfonic acid, or p-toluene sulfonic acid, and the metal catalyst may be, for example, an organometal catalyst, a metal oxide catalyst, a metal salt catalyst or a metal itself.

The metal component may be, for example, any one selected from the group consisting of tin, titanium and zirconium, or a mixture of two or more thereof.

In addition, a step for removing unreacted alcohols and reaction by-products by distillation may be further included after the transesterification. The distillation may be, for example, a two-step distillation by which the alcohols and the by-products are individually separated using the difference of the boiling points. As another example, the distillation may be mixture distillation. In this case, the effects of stably securing an ester-based plasticizer composition in a desired composition ratio can be achieved. The mixture distillation means distillation of the unreacted alcohols and the by-products simultaneously.

According to another embodiment of the present invention, a resin composition including the plasticizer composition described above and a resin is provided.

The resin may be one well-known in the art. For example, a mixture of one or more selected from, but not limited to, the group consisting of a straight vinyl chloride polymer, a paste vinyl chloride polymer, an ethylene vinyl acetate copolymer, an ethylene polymer, a propylene polymer, polyketone, polystyrene, polyurethane, natural rubbers, and synthetic rubbers may be used.

The plasticizer composition may be included in an amount of 5 to 150 parts by weight, preferably, 5 to 130 parts by weight, or 10 to 120 parts by weight based on 100 parts by weight of the resin.

Generally, the resin for which the plasticizer composition is used can be prepared into a resin product through melt processing or plastisol processing, and a resin prepared by melt processing and a resin prepared by plastisol processing may be produced differently according to each polymerization method.

For example, in the case of using a vinyl chloride polymer in melt processing, solid-phase resin particles having a large average particle diameter are prepared by suspension polymerization or the like and used, and the vinyl chloride polymer is a straight vinyl chloride polymer. In the case of using a vinyl chloride polymer in plastisol processing, a sol state resin in which minute resin particles are distributed is prepared by emulsion polymerization or the like and used, and the vinyl chloride polymer is a paste vinyl chloride resin.

In the case of the straight vinyl chloride polymer, a plasticizer may be included in a range of 5 to 150 parts by

weight, preferably, 5 to 80 parts by weight based on 100 parts by weight of the polymer. In the case of the paste vinyl chloride polymer, the plasticizer may be included in a range of 5 to 150 parts by weight, preferably, 40 to 120 parts by weight based on 100 parts by weight of the polymer.

The resin composition may further include a filler. The filler may be present in an amount of 0 to 300 parts by weight, preferably, 50 to 200 parts by weight, more preferably, 100 to 200 parts by weight based on 100 parts by weight of the resin.

The filler may be a well-known filler in the art and is not particularly limited. For example, the filler may be a mixture of one or more selected from silica, magnesium carbonate, calcium carbonate, hard coal, talc, magnesium hydroxide, titanium dioxide, magnesium oxide, calcium hydroxide, aluminum hydroxide, aluminum silicate, magnesium silicate, and barium sulfate.

In addition, the resin composition may further include other additives, such as a stabilizer, as needed. Each of the other additives, such as a stabilizer, may be included, for example, in an amount of 0 to 20 parts by weight, and preferably 1 to 15 parts by weight based on 100 parts by weight of the resin.

The stabilizer may be, for example, but is not particularly limited to, a calcium-zinc-based (Ca-Zn-based) stabilizer such as a composite stearate of calcium-zinc, or a barium-zinc-based (Ba-Zn-based) stabilizer.

The resin composition can be applied to both melt processing and plastisol processing as described above. For example, calendering processing, extrusion processing, or injection processing can be applied for melt processing, and coating processing, or the like can be applied for plastisol processing.

## Examples

Hereinafter, the present invention will be described in more detail by examples. Examples according to the present invention may be modified into various other types, and the scope of the present invention should not be limited to examples described below. The examples of the present invention are provided for completely explaining the present invention to a person having an average knowledge in the art.

### Example 1

To a reactor equipped with a stirrer, a condenser and a decanter, 500 g of citric acid anhydride, 433 g of n-butanol, 600 g of n-heptanol (the molar ratio of the two alcohols is 5:5), and 2 g of tetrabutyl titanate (TnBT) were introduced and esterification was carried out under a nitrogen atmosphere. The reaction was completed and unreacted alcohols were removed. Then, the catalyst and the composition were neutralized and washed with an alkaline solution. A purification process of removing unreacted alcohols and water was carried out to obtain the composition of Example 1 containing tri(n-butyl) citrate, di(n-butyl)(n-heptyl) citrate, di(n-heptyl)(n-butyl) citrate, and tri(n-heptyl) citrate in an amount of 11.5 wt%, 36.4 wt%, 39.2 wt%, and 12.9 wt%, respectively.

### Examples 2 to 9

To a reactor equipped with a stirrer, a condenser and a decanter, 500 g of citric acid anhydride, 1,180 g of n-heptanol, and 2 g of tetrabutyl titanate (TnBT) were introduced and esterification was carried out under a nitrogen atmosphere. The reaction was completed and unreacted alcohols were removed. Then, 253 g of n-butanol was introduced to perform transesterification. After the reaction was completed, the catalyst and the composition were neutralized. A purification process of removing unreacted alcohols and water was carried out to finally obtain the composition of Example 2 containing tri(n-butyl) citrate, di(n-butyl)(n-heptyl) citrate, di(n-heptyl)(n-butyl) citrate, and tri(n-heptyl) citrate in an amount of 0.7 wt%, 8.9 wt%, 38.2 wt%, and 52.2 wt%, respectively.

In the above reaction, the types and introduction amounts of reactants were adjusted to prepare the compositions of Examples 3 to 9 having the compositions as shown in Table 1 below.

### Comparative Example 1

Dioctyl phthalate (DOP, LG Chemical, LTD.) was used as a plasticizer.

### Comparative Example 2

Diisononyl phthalate (DINP, LG Chemical, LTD.) was used as a plasticizer.

**Comparative Example 3**

Di(2-ethylhexyl) terephthalate (GL300, LG Chemical, LTD.) was used as a plasticizer.

**Comparative Examples 4 and 6**

The same reaction as in Example 1 was carried out except for using only 2-ethyl hexanol instead of 2-ethyl hexanol and n-butanol as the alcohols in Example 1 to obtain tri(2-ethyl hexyl) citrate of Comparative Example 4.
In Comparative Examples 5 and 6, the alcohol in Comparative Example 4 was changed to the alcohols shown in Table 1 below.

**Comparative Examples 7 and 14**

The same reaction as in Example 6 was carried out except for changing the alcohols used for direct esterification and the higher alcohols and lower alcohols used for transesterification to those shown in Table 1 below, respectively, to obtain citrate compositions.

[Table 1]

| Classificat ion | Lower alcoh ols | Highe r alcoh ols | Introduc tion amount of alcohols | Product composition | | | |
|---|---|---|---|---|---|---|---|
| | | | | Lowe r non-hybr id | Lower hybri d | High er hybr id | High er non-hybr id |
| Example 1 | n-B | n-Hp | 5:5 | 11.5 | 36.4 | 39.2 | 12.9 |
| Example 2 | n-B | n-Hp | C4 20% of TnHpC | 0.7 | 8.9 | 38.2 | 52.2 |
| Example 3 | n-B | 5-MHx | C4 19% of TiHpC | 1.4 | 13.6 | 41.9 | 43.1 |
| Example 4 | n-B | n-Hp | C4 30% of TnHpC | 2.7 | 22.5 | 40.7 | 34.1 |
| Example 5 | n-B | n-Hp | C4 40% of TnHpC | 5.3 | 30.2 | 38.5 | 26.0 |
| Example 6 | n:i=9 :1 | n-Hp | C4 30% of TnHpC | 2.5 | 21.0 | 41.2 | 35.3 |
| Example 7 | n:i=7 :3 | n:5=9 :1 | C4 30% of TnHpC | 2.0 | 20.3 | 40.8 | 36.9 |
| Example 8 | iB | n-Hp | C4 20% of TnHpC | 1.0 | 8.5 | 39.8 | 50.7 |
| Example 9 | n-B | n-Hp | C7 50% of TnBC | 18.5 | 38.5 | 36.2 | 6.8 |
| Comparative Example 4 | 2-EH | | - | - | - | - | - |
| Comparative Example 5 | n-P | | - | - | - | - | - |
| Comparative Example 6 | n-Hp | | - | - | - | - | - |
| Comparative Example 7 | n-B | 2-EH | C4 20% of TEHC | 3.2 | 19.7 | 42.8 | 34.3 |
| Comparative Example 8 | n-B | 2-EH | C8 30% of TBC | 26.7 | 42.5 | 24.3 | 6.5 |
| Comparative Example 9 | n-B | 2-EH | C4 40% of TEHC | 11.0 | 32.2 | 42.3 | 14.5 |
| Comparative Example 10 | n-B | IN | C4 20% of TINC | 2.8 | 18.2 | 44.0 | 35.0 |
| Comparative Example 11 | n-P | IN | C5 20% of TINC | 3.0 | 18.5 | 44.1 | 34.4 |
| Comparative Example 12 | n-Hx | 2-EH | C6 30% of TEHC | 5.2 | 27.0 | 44.7 | 23.1 |

(continued)

| Classificat ion | Lower alcoh ols | Highe r alcoh ols | Introduc tion amount of alcohols | Product composition | | | |
|---|---|---|---|---|---|---|---|
| | | | | Lowe r non-hybr id | Lower hybri d | High er hybr id | High er non-hybr id |
| Comparative Example 13 | n-P | n-Hp | C5 20% of THpC | 2.4 | 17.5 | 43.2 | 36.9 |

- n-B: n-butanol
- iB: isobutanol
- n-Hp: n-heptanol
- 5-MHx: 5-methyl hexanol
- n-Hx: n-hexanol
- 2-EH: 2-Ethyl hexanol
- IN: isononanol
- In Table 1 above, "C4 20% of TnHpC" means 20 parts by weight of the C4 alcohol based on 100 parts by weight of TnHpC and similar descriptions can be construed equally.

**Experimental example 1: Evaluation of sheet performance**

By using the plasticizers of Examples and Comparative Examples, specimens were manufactured according to the formulation and manufacturing conditions below and ASTM D638.
**(1) Formulation:** 100 parts by weight of a straight vinyl chloride polymer (LS100S), 30 parts by weight of a plasticizer, and 3 parts by weight of a stabilizer (BZ-153T)
**(2) Mixing:** mixing at 98°C in 700 rpm
**(3) Manufacture of specimen:** 1T, 2T, and 3T sheets were manufactured by processing at 160°C for 4 minutes using a roll mill, and at 180°C for 2.5 minutes (low pressure) and 2 minutes (high pressure) using a press.
**(4) Evaluation items**

1) Hardness: Shore hardness (Shore "A" and "D") at 25°C was measured using a 3T specimen for 10 seconds using ASTM D2240. It is evaluated that the smaller the value is, the better the plasticization efficiency is.
2) Tensile strength: By ASTM D638 method, a specimen was drawn in a cross-head speed of 200 mm/min using a test apparatus of U.T.M (manufacturer: Instron, model name: 4466), and a point where the 1T specimen was cut was measured. The tensile strength was calculated as follows:

$$\text{Tensile strength (kgf/cm}^2\text{)} = \text{load value (kgf)} / \text{thickness (cm)} \times \text{width (cm)}$$

3) Measurement of elongation rate: "Elongation rate" was measured according to ASTM D638. After pulling at a cross head speed of 200 mm/min by using the U.T.M, a position where the 1T specimen was cut was measured, and the elongation rate was calculated as follows:

$$\text{Elongation rate (\%)} = \text{length after elongation} / \text{initial length} \times 100.$$

4) Measurement of migration loss: According to KSM-3156, a specimen with a thickness of 2 mm or more was obtained, and glass plates were attached onto both sides of the 1T specimen and a load of 1 kgf/cm$^2$ was applied. The specimen was stood in a hot air circulation oven (80°C) for 72 hours and was taken out and cooled at room temperature for 4 hours. After that, the glass plates attached onto both sides of the specimen are removed, and the weights of the glass plates and the specimen plate before and after being left standing in the oven are measured. The migration loss was calculated by the following equation.

Migration loss (%) = {[(initial weight of specimen at room temperature) - (weight of specimen after being left standing in oven)] / (initial weight of specimen at room temperature)} x 100

5) Measurement of volatile loss: The specimen manufactured was processed at 80°C for 72 hours, and the weight of the specimen was measured.
The volatile loss was calculated as follows:

Volatile loss (wt %) = {[(weight of initial specimen) - (weight of specimen after processing)] / (weight of initial specimen)} x 100

6) Stress test (stress resistance): A specimen having a thickness of 2 mm was left in a bent state at 23°C for 168 hours, and then the degree of migration (the degree of oozing) was observed, and the result was shown as a numerical value. The numerical value closer to 0 represented the excellent property.
7) Measurement of elongation retention (%): The measurement of elongation retention was performed by applying heat at 100°C for 168 hours and measuring the remaining elongation rate of a specimen. The measurement method is the same as that of the elongation rate.

**(5) Evaluation results**

The evaluation results on the test items are shown in Tables 2 and 3 below.

[Table 2]

| | Hard ness (Sho re "A") | Hard ness (Sho re "D") | Migr atio n loss (%) | Volatile loss (%) | | Tensi le stren gth (kgf/ cm$^2$) | Elong ation rate (%) | Elon gati on rete ntio n (%) |
|---|---|---|---|---|---|---|---|---|
| | | | | 80°C | 100°C | | | |
| Example 1 | 83.0 | 36.2 | 0.67 | 1.14 | 1.85 | 183.7 | 325.6 | 91.2 |
| Example 2 | 85.7 | 38.5 | 1.23 | 0.70 | 1.27 | 187.7 | 299.4 | 92.1 |
| Example 3 | 86.1 | 39.0 | 1.13 | 0.80 | 1.44 | 186.3 | 303.5 | 92.8 |
| Example 4 | 84.6 | 37.5 | 1.02 | 0.89 | 1.42 | 188.0 | 305.6 | 92.4 |
| Example 5 | 83.9 | 37.0 | 0.96 | 0.95 | 1.50 | 186.4 | 310.4 | 91.3 |
| Example 6 | 84.7 | 37.5 | 1.00 | 0.76 | 1.30 | 190.5 | 309.7 | 93.6 |
| Example 7 | 84.6 | 37.3 | 0.95 | 0.70 | 1.20 | 192.3 | 308.6 | 95.7 |
| Example 8 | 85.5 | 38.5 | 0.82 | 0.52 | 0.88 | 186.9 | 305.5 | 98.6 |
| Example 9 | 80.1 | 34.1 | 0.68 | 1.35 | 1.86 | 183.5 | 326.1 | 92.6 |

(continued)

| | Hard ness (Sho re "A") | Hard ness (Sho re "D") | Migr atio n loss (%) | Volatile loss (%) | | Tensi le stren gth (kgf/ $cm^2$) | Elong ation rate (%) | Elon gati on rete ntio n (%) |
|---|---|---|---|---|---|---|---|---|
| | | | | 80°C | 100° C | | | |
| Comparative Example 1 | 84.6 | 38.3 | 1.31 | 1.26 | 3.10 | 180.0 | 285.6 | 90.5 |
| Comparative Example 2 | 86.9 | 40.6 | 2.08 | 0.71 | 1.29 | 197.8 | 290.3 | 93.6 |
| Comparative Example 3 | 88.4 | 41.7 | 5.91 | 0.81 | 1.47 | 197.5 | 310.8 | 92.7 |
| Comparative Example 4 | 90.5 | 43.6 | 1.92 | 0.21 | 0.56 | 205.6 | 301.2 | 95.8 |
| Comparative Example 5 | 82.8 | 36.0 | 1.10 | 1.84 | 3.02 | 175.8 | 287.5 | 93.1 |
| Comparative Example 6 | 88.7 | 41.7 | 1.45 | 1.24 | 2.35 | 187.6 | 295.3 | 93.5 |
| Comparative Example 7 | 85.5 | 39.2 | 1.75 | 1.65 | 3.47 | 175.3 | 284.3 | 84.2 |
| Comparative Example 8 | 81.2 | 34.6 | 1.46 | 3.58 | 6.28 | 165.2 | 265.8 | 78.5 |
| Comparative Example 9 | 83.8 | 37.0 | 1.60 | 2.74 | 5.82 | 172.1 | 280.0 | 81.0 |
| Comparative Example 10 | 88.9 | 41.6 | 2.48 | 1.58 | 2.66 | 180.2 | 288.3 | 95.2 |
| Comparative Example 11 | 91.3 | 43.9 | 2.33 | 0.84 | 1.25 | 182.6 | 295.1 | 96.1 |
| Comparative Example 12 | 87.9 | 41.1 | 2.08 | 0.90 | 1.33 | 191.2 | 274.0 | 93.4 |
| Comparative Example 13 | 86.2 | 40.2 | 1.56 | 1.84 | 3.66 | 169.4 | 270.8 | 82.4 |

[Table 3]

| | Stress resistance | | |
|---|---|---|---|
| | One day elapsed | Two days elapsed | Three days elapsed |
| Example 1 | 0 | 0 | 0 |
| Example 2 | 0.5 | 0.5 | 0.5 |
| Example 3 | 0.5 | 1.5 | 1.0 |
| Example 4 | 0.5 | 0.5 | 0.5 |
| Example 5 | 0 | 0.5 | 0.5 |
| Example 6 | 0.5 | 0.5 | 0.5 |
| Example 7 | 0.5 | 0.5 | 0.5 |
| Example 8 | 0 | 0.5 | 0.5 |
| Example 9 | 0 | 0 | 0 |

(continued)

| | Stress resistance | | |
|---|---|---|---|
| | One day elapsed | Two days elapsed | Three days elapsed |
| Comparative Example 1 | 0.5 | 1.0 | 0 |
| Comparative Example 2 | 1.0 | 1.5 | 1.0 |
| Comparative Example 3 | 1.5 | 3.0 | 3.0 |
| Comparative Example 4 | 1.0 | 1.5 | 2.5 |
| Comparative Example 5 | 0.5 | 1.0 | 1.0 |
| Comparative Example 6 | 1.0 | 2.0 | 2.0 |
| Comparative Example 7 | 0.5 | 0.5 | 1.0 |
| Comparative Example 8 | 0.5 | 0.5 | 0.5 |
| Comparative Example 9 | 0.5 | 0.5 | 0.5 |
| Comparative Example 10 | 1.0 | 2.0 | 2.5 |
| Comparative Example 11 | 1.5 | 2.5 | 3.0 |
| Comparative Example 12 | 1.0 | 1.5 | 2.0 |
| Comparative Example 13 | 0.5 | 0.5 | 1.0 |

Referring to Tables 2 and 3 above, Examples 1 to 9 to which the plasticizer composition according to an embodiment of the present invention was applied, showed significantly improved plasticization efficiency and migration loss compared to Comparative Examples 1 to 3, which are used as conventional products. It can be found that Examples 1 to 9 also showed excellent elongation retention and stress resistance.

Also, Comparative Examples 4 to 6 are obtained by applying the citrate prepared from one type of alcohol without hybridizing the alkyl groups of the citrate with two or more alcohols. Comparative Examples 4 to 6 were found to be inferior in all physical properties compared to Examples 1 to 9, and it can be seen that there is a problem in that the physical properties vary greatly depending on the number of carbon atoms.

Meanwhile, Comparative Examples 7 to 13 are obtained by hybridizing the alkyl groups of two types of alcohols, like Examples according to the present invention, but not adjusting the number of carbon atoms to C4 for the lower alcohols and C7 for the higher alcohols.

Looking at these results, in Comparative Examples 7 to 9 and 13, remarkably poor tensile strength and elongation rate were shown and the elongation retention was very poor. Therefore, it can be confirmed that the flexibility is greatly lost in a high-temperature environment, and the deterioration of the volatile loss can also be confirmed.

Also, it can be found that although Comparative Examples 11 and 12 have high hardness and thus poor plasticization efficiency, the migration resistance and stress resistance are also poor.

Thus, when applying citrate as a plasticizer as in Examples of the present invention, it could be found that: it is necessary to use two or more alcohols, but to use C4 and C7 alcohols in combination, thereby hybridizing the alkyl groups of the citrate; and in that case, implementation of a plasticizer having excellent performance is possible.

## Claims

**1.** A plasticizer composition comprising a citrate-based composition containing three or more citrates represented by the following Formula 1,

wherein an alkyl group of the citrate is derived from a C4 alcohol and a C7 alcohol,
the C4 alcohol includes one or more selected from the group consisting of n-butanol and iso-butanol, and
the C7 alcohol includes one or more selected from n-heptyl group or a branched heptyl group:

[Formula 1]

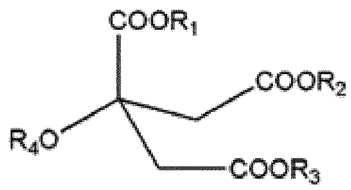

wherein $R_1$ to $R_3$ are each independently an alkyl group having 4 or 7 carbon atoms, and $R_4$ is hydrogen or an acetyl group.

**2.** The plasticizer composition of claim 1, wherein the citrate-based composition comprises: a lower alkyl-based citrate including a lower non-hybrid citrate having a C4 alcohol-derived alkyl group, and a lower hybrid citrate having a C4 alcohol-derived alkyl group and a C7 alcohol-derived alkyl group, in which the C4 alcohol-derived alkyl groups are more than the C7 alcohol-derived alkyl groups; and a higher alkyl-based citrate including a higher hybrid citrate having a C4 alcohol-derived alkyl group and a C7 alcohol-derived alkyl group, in which the C7 alcohol-derived alkyl groups are more than the C4 alcohol-derived alkyl groups, and a higher non-hybrid citrate having a C7 alcohol-derived alkyl group.

**3.** The plasticizer composition of claim 1, wherein the molar ratio of the C4 alcohol to the C7 alcohol is 90:10 to 10:90.

**4.** The plasticizer composition of claim 1, wherein the molar ratio of the C4 alcohol to the C7 alcohol is 60:40 to 10:90.

**5.** The plasticizer composition of claim 1, wherein the C7 alcohol includes n-heptanol.

**6.** The plasticizer composition of claim 1, wherein the C7 alcohol includes n-heptanol and at least one branched alcohol selected from 4-methyl hexanol and 5-methyl hexanol, and n-heptanol is included in an amount of 50 wt% or more based on the total weight of the C7 alcohol.

**7.** The plasticizer composition of claim 2, wherein the weight ratio of the lower alkyl-based citrates to the higher alkyl-based citrates is 80:20 to 1:99.

**8.** The plasticizer composition of claim 2, wherein the weight ratio of the sum of the non-hybrid citrates to the sum of the hybrid citrates is 80:20 to 5:95.

**9.** A resin composition comprising: 100 parts by weight of a resin; and 5 to 150 parts by weight of the plasticizer composition of claim 1.

**10.** The resin composition of claim 9, wherein the resin is one or more selected from the group consisting of a straight vinyl chloride polymer, a paste vinyl chloride polymer, an ethylene vinyl acetate copolymer, an ethylene polymer, a propylene polymer, polyketone, polystyrene, polyurethane, natural rubbers, and synthetic rubbers.

## INTERNATIONAL SEARCH REPORT

International application No.
**PCT/KR2021/000388**

**A. CLASSIFICATION OF SUBJECT MATTER**

**C08K 5/11**(2006.01)i; **C08K 5/00**(2006.01)i; **C08L 101/00**(2006.01)i

According to International Patent Classification (IPC) or to both national classification and IPC

**B. FIELDS SEARCHED**

Minimum documentation searched (classification system followed by classification symbols)

C08K 5/11(2006.01); C07C 67/02(2006.01); C07C 69/704(2006.01); C08K 3/00(2006.01); C08K 5/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 가소제(plasticizer), 시트레이트(citrate), C4 알코올(C4 alcohol), C7 알코올(C7 alcohol), 수지 조성물(resin composition)

**C. DOCUMENTS CONSIDERED TO BE RELEVANT**

| Category* | Citation of document, with indication, where appropriate, of the relevant passages | Relevant to claim No. |
|---|---|---|
| X | KR 10-2017-0020282 A (LG CHEM, LTD.) 22 February 2017 (2017-02-22)<br>See claims 1, 4 and 15-20; paragraphs [0001], [0090]-[0093], [0121], [0139]-[0141], [0144], [0163], [0164] and [0167]; and manufacturing examples 1 and 2. | 1-10 |
| X | KR 10-2002-0085812 A (CELANESE CHEMICALS EUROPE GMBH) 16 November 2002 (2002-11-16)<br>See claims 1-7, 16 and 17; and pages 2 and 3. | 1-8 |
| A | US 2011-0046283 A1 (GRASS, M. et al.) 24 February 2011 (2011-02-24)<br>See abstract; and claims 1-5 and 11-18. | 1-10 |
| A | CN 103113617 A (SHANDONG ONE TW POLYMER MATERIALS CO., LTD.) 22 May 2013 (2013-05-22)<br>See claims 1-8. | 1-10 |
| A | KR 10-2016-0095875 A (HANWHA CHEMICAL CORPORATION) 12 August 2016 (2016-08-12)<br>See claims 1-3 and 6. | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 April 2021** | **15 April 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.
**PCT/KR2021/000388**

| Patent document cited in search report | Publication date (day/month/year) | Patent family member(s) | Publication date (day/month/year) |
|---|---|---|---|
| KR 10-2017-0020282 A | 22 February 2017 | CN 106795325 A | 31 May 2017 |
| | | CN 111732756 A | 02 October 2020 |
| | | CN 111732757 A | 02 October 2020 |
| | | CN 111777796 A | 16 October 2020 |
| | | DE 202016008699 U1 | 13 February 2019 |
| | | EP 3130631 A1 | 15 February 2017 |
| | | EP 3130631 A4 | 12 July 2017 |
| | | EP 3130631 B1 | 01 April 2020 |
| | | EP 3670592 A1 | 24 June 2020 |
| | | EP 3670593 A1 | 24 June 2020 |
| | | ES 2788733 T3 | 22 October 2020 |
| | | KR 10-1674317 B1 | 08 November 2016 |
| | | KR 10-1881646 B1 | 24 July 2018 |
| | | KR 10-2004732 B1 | 29 July 2019 |
| | | KR 10-2016-0099453 A | 22 August 2016 |
| | | KR 10-2016-0130363 A | 11 November 2016 |
| | | KR 10-2019-0024935 A | 08 March 2019 |
| | | KR 10-2019-0024936 A | 08 March 2019 |
| | | KR 10-2020-0031588 A | 24 March 2020 |
| | | KR 10-2079234 B1 | 19 February 2020 |
| | | KR 10-2197487 B1 | 31 December 2020 |
| | | TW 201700568 A | 01 January 2017 |
| | | TW I617605 B | 11 March 2018 |
| | | US 2017-0081501 A1 | 23 March 2017 |
| | | WO 2016-129876 A1 | 18 August 2016 |
| KR 10-2002-0085812 A | 16 November 2002 | DE 10122145 A1 | 28 November 2002 |
| | | EP 1256566 A2 | 13 November 2002 |
| | | EP 1256566 A3 | 24 September 2003 |
| | | PL 353721 A1 | 18 November 2002 |
| | | US 2002-0198402 A1 | 26 December 2002 |
| US 2011-0046283 A1 | 24 February 2011 | DE 102008002168 A1 | 10 December 2009 |
| | | EP 2297083 A2 | 23 March 2011 |
| | | EP 2297083 B1 | 24 December 2014 |
| | | EP 2857383 A1 | 08 April 2015 |
| | | TW 201004918 A | 01 February 2010 |
| | | TW I461404 B | 21 November 2014 |
| | | US 8431638 B2 | 30 April 2013 |
| | | WO 2009-146991 A2 | 10 December 2009 |
| | | WO 2009-146991 A3 | 04 February 2010 |
| CN 103113617 A | 22 May 2013 | CN 103113617 B | 10 December 2014 |
| KR 10-2016-0095875 A | 12 August 2016 | CN 107428989 A | 01 December 2017 |
| | | EP 3255086 A1 | 13 December 2017 |
| | | EP 3255086 A4 | 18 July 2018 |
| | | JP 2018-502981 A | 01 February 2018 |
| | | JP 6469256 B2 | 13 February 2019 |
| | | KR 10-1845338 B1 | 04 April 2018 |
| | | WO 2016-126080 A1 | 11 August 2016 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020200005440